# EUROPEAN PATENT SPECIFICATION

(11) **EP 1 514 866 B1**
(45) Date of publication and mention of the grant of the patent: **15.11.2006**
(21) Application number: 04022599.7
(22) Date of filing: 23.09.1999
(51) Int. Cl.: C07C 253/30, C07C 255/57

(54) **Process for producing cyanobenzoic acid derivatives**
Verfahren zur Herstellung von Derivaten der Cyanobenzoesäure
Procédé de préparation de dérivés de l'acide cyano-benzoique

(30) Priority: 24.09.1998 JP 27021398; 09.10.1998 JP 28779698; 14.10.1998 JP 29196398; 23.10.1998 JP 30296098; 23.10.1998 JP 30296198; 17.11.1998 JP 32621198; 18.11.1998 JP 32817198; 15.04.1999 JP 10736599; 21.04.1999 JP 11362199
(43) Date of publication of application: 16.03.2005
(62) Divisional of application: 99118800.4
(73) Proprietor: SHOWA DENKO KABUSHIKI KAISHA, Minato-ku, Tokyo (JP)
(72) Inventor: Yasuda, Hiroshi Kawasaki Research Laboratory, Kawasaki-k Kawasaki-shi Kanagawa210-0858 (JP); Ito, Haruaki, Minato-ku Tokyo 105-8518 (JP); Tani, Takashi Kawasaki Works, Showa Denko K.K., Kawasaki-shi Kanagawa 210-0865 (JP); Ohshiro, Kimitaka Kawasaki Research Laboratory, Kawasaki-k Kawasaki-shi Kanagawa210-0858 (JP); Saito, Makoto Kawasaki Works, Showa Denko K.K., Kawasaki-shi Kanagawa 210-0865 (JP); Soya, Sumio, Minato-ku Tokyo 105-8518 (JP); Marumo, Kuniomi, Tokyo 157-0072 (JP)
(74) Representative: Strehl Schübel-Hopf & Partner

(56) References cited:
- EP-A- 0 518 412

## Description

### FIELD OF THE INVENTION

The present invention relates to a process for producing a cyanobenzoyl chloride compound by use of a phthalonitrile compound as a raw material.

The cyanobenzoyl chloride compound which is produced through the process according to the present invention is useful intermediates for a variety of chemicals such as pharmaceuticals, agrochemicals, liquid crystals, and monomers for functional polymers.

### BACKGROUND OF THE INVENTION

Conventional processes for producing a cyanobenzoic chloride compound will be described.

p-Cyanobenzoyl chloride is synthesized through reaction of p-cyanobenzoic acid with a chlorinating agent. Examples of chlorinating agents include thionyl chloride (JP-B-1-31501, Johan Kamphuis, *et al.,* J. Chem. Soc. Perkin Trans., 2 (1987) 907), oxalyl chloride (Robert J. Weikert, *et al.,* J. Med. Chem., 34 (1991) 1630), and phosphorus pentachloride (Raffaello Fusco, *et al.,* Ann. Chim. (Rome), 42 (1952) 94). As an alternative to chlorination to produce an acid chloride, JP-A-63-313761 discloses a process involving reaction of 4-trichloromethylbenzamide in the presence of ferric chloride as a catalyst, and JP-A-52-39649 discloses a process involving reaction of a terephthalamate salt and phosphorus oxychloride.

Conventional processes for chlorinating a p-cyanobenzoic acid compound to produce the corresponding acid chloride also involve problems. When thionyl chloride is used, sulfur dioxide which is generated must be separated for detoxification, which is not economical. When oxalyl chloride is used, highly toxic carbon monoxide produced therefrom must be separated for detoxification, which is not economical. When phosphorus pentachloride is used, a compound produced from the reaction must be removed in order to prevent eutrophication of lakes, ponds, and rivers, and such an excess step for the sake of environmental safety disturbs practical application of phosphorous pentachloride. Terephthalamate salt and 4-trichloromethylbenzamide, serving as raw materials for a process involving reaction therebetween, are expensive and are not easily available, to thereby make the process economically disadvantageous.

As described above, conventional processes for producing a p-cyanobenzoic chloride compound involve problems such as dangerous steps for separation and removal of by-products, and poor availability of raw materials.

### SUMMARY OF THE INVENTION

In view of the foregoing, an object of the present invention is to provide a process for producing a cyanobenzoyl chloride compound with high selectivity and yield in an industrially advantageous manner.

The present inventors have conducted earnest studies, and they are able to satisfy the above objects.

In the present invention, there is provided a process for producing a cyanobenzoyl chloride compound represented by the following formula: 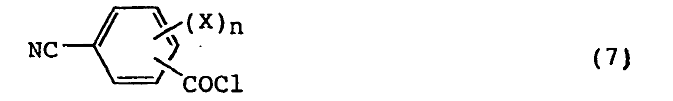 wherein each of -COCl and -X represents a substituent of the benzene ring; the -COCl group is bonded at the m- or the p-position with respect to the CN group; X represents a chlorine atom or a fluorine atom; n is an integer between 0 and 4 inclusive; and when n is 2 or more the plurality of X's may be identical to or different from one another,
which process comprises reacting a cyanobenzoic acid compound represented by the following formula: 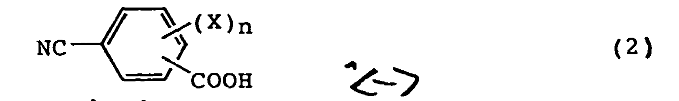 wherein -X represents a substituent of the benzene ring with phosgene or a precursor thereof, the precursor being a substance convertible to phosgene with heat or through a treatment with a catalyst.

Preferably, the above-described reaction is carried out in the presence of a cyanobenzoyl chloride compound corresponding to the cyanobenzoic acid compound.

Preferably, the above-described reaction is carried out in the presence of a tertiary amide compound or a tertiary amine compound.

Preferably, the cyanobenzoic acid compound represented by formula (2) is m- or p-cyanobenzoic acid, and the cyanobenzoyl chloride compound represented by formula (7) is m- or p-cyanobenzoyl chloride.

In a preferred embodiment, the process according to the present invention is conducted using phosgene in an amount of 1.1 to 4 mol per 1 mol of the cyanobenzoic acid compound.

### DETAILED DESCRIPTION

### Process for producing a cyanobenzoyl chloride compound from a cyanobenzoic acid compound serving as a raw material

This process comprises reacting a cyanobenzoic acid compound with phosgene or a precursor thereof and stirring the reaction mixture at a predetermined temperature for a predetermined period of time. In order to promote the reaction, an additive such as a cyanobenzoyl chloride compound corresponding to the cyanobenzoic acid compound, a tertiary amide compound, or a tertiary amine compound may be incorporated into the reaction system in the presence of an organic solvent.

The cyanobenzoic acid compound used in the process will next be described. Examples of unsubstituted cyanobenzoic acid compounds include p-cyanobenzoic acid and m-cyanobenzoic acid. These compounds may be synthesized through a known method or through the process of the present invention.

The halo-substituted cyanobenzoic acid compound will next be described. Chlorinated cyanobenzoic acid compounds such as 4-cyano-2,3,5,6-tetrachlorobenzoic acid and 3-cyano-2,4,5,6-tetrachlorobenzoic acid may be synthesized from a chlorinated terephthalonitrile compound such as tetrachloroterephthalonitrile or a chlorinated isophthalonitrile compound such as tetrachloroisophthalonitrile, through a known method or through the process of the present invention.

Fluorinated cyanobenzoic acid compounds such as 4-cyano-2,3,5,6-tetrafluorobenzoic acid and 3-cyano-2,4,5,6-tetrafluorobenzoic acid may be synthesized through a known method or through the process of the present invention, by use of a fluorinated tetrephthalonitrile compound such as tetrafluoroterephthalonitrile or a fluorinated isophthalonitrile compound such as tetrafluoroisophthalonitrile, which is obtained through fluorination of the corresponding chlorinated species.

Phosgene or a precursor thereof used in the process will next be described. Phosgene may be introduced to the reaction system in the form of a gas or liquid produced through pressurization or cooling.

The phosgene gas is used in an amount of at least equiomol based on the cyanobenzoic acid compound, preferably 1.1-4 mol based on 1 mol of the cyanobenzoic acid compound, more preferably, 1.1-1.5 mol, with unreacted phosgene gas being recycled.

The reaction between phosgene and a cyanobenzoic acid compound will next be described in detail. When a phosgene monomer is employed, the monomer is reacted directly with a cyanobenzoic acid compound. As the reaction proceeds, carbon dioxide and hydrochloric acid are formed during formation of the corresponding cyanobenzoyl chloride compound. Phosgene is typically introduced into the reaction system until the phosgene is no longer consumed and formation of carbon dioxide and hydrochloric acid is completed. A precursor of phosgene is easily converted to phosgene with heat or through a treatment with a catalyst. Active carbon may be used as the catalyst. Examples of precursors include a dimer and a trimer of phosgene. The phosgene dimer may directly be introduced to a reactor. Alternatively, the dimer may be converted to phosgene gas in another reactor, and the gas formed may be introduced to the reactor. The phosgene trimer, which is solid at room temperature, may be fed to the reactor in a required amount in one portion or may be fed to the reactor in the form of a solution after dissolution in an inert solvent.

A method for isolating the formed cyanobenzoyl chloride compound will next be described. In accordance with needs, excessive phosgene and hydrochloric acid remaining in the reaction mixture are removed through a method such as heating or bubbling of an inert gas; e.g., nitrogen. When a solvent is used, the solvent is removed through distillation. The cyanobenzoyl chloride compound formed can be isolated from the reaction mixture through a customary method, such as distillation or crystallization.

In general, a cyanobenzoic acid compound has poor solubility to an organic solvent. Therefore, a cyanobenzoyl chloride compound corresponding to the cyanobenzoic acid compound is added during the reaction, or is used as a solvent, to thereby enhance reaction efficiency. In this case, the cyanobenzoyl chloride compound and the cyanobenzoic acid compound may be mixed with each other directly, or with an inert solvent when stirring is difficult. The cyanobenzoyl chloride compound provides an effect of promoting the reaction.

The process employs an aprotic organic solvent. Examples of such organic solvents include nitriles such as acetonitrile; tertiary formamides such as dimethylformamide and diethylformamide; sulfur-containing compounds such as sulfolane; imidazolidones such as 1,3-dimethyl-2-imidazolidone; ethers such as dioxane, 1,2-dimethoxyethane, and diglyme; halohydrocarbons such as dichloromethane, chloroform, and 1,2-dichloroethane; and aromatic hydrocarbons such as benzene, toluene, and o-, m-, and p-xylene, and mixed xylene. These organic solvents may be used alone or as a combination of two or more.

The reaction time is preferably 10 minutes to 10 hours, depending on the composition of the employed solvent.

The process may employ additives so as to promote reaction. Examples of such additives include tertiary formamides such as dimethylformamide, diethylformamide, dimethylpropionamide, and dibutylformamide. When used, the amount of tertiary formamide is at least 0.01 mol equivalents based on the carboxylic acid.

A tertiary amine compound may be added as a reaction promoter. The tertiary amine compound functions as a catalyst for activating phosgene or as a scavenger of hydrochloric acid formed during transformation of a cyanobenzoic acid to its acid chloride. Preferred examples of the tertiary amine compound include aliphatic tertiary amines such as trimethylamine, triethylamine, and tribenzylamine; aromatic tertiary amines such as triphenylamine and tri-p-toluylamine; and heterocyclic compounds such as imidazole, pyridine, p-dimethylaminopyridine, N-methylmorpholine, quinoline, isoquinoline, pyrimidine, and piperazine. When used as a catalyst for activating phosgene, the tertiary amine compound is used in an amount of at least 0.01 mol equivalents based on the carboxylic acid. When the amine compound is used for activating phosgene and as a scavenger of hydrochloric acid, the amine compound must be used in an amount of at least equimol to phosgene employed in the reaction.

### EXAMPLES

The present invention will next be described in more detail by way of examples, which should not be construed as limiting the invention thereto. Unless otherwise indicated, all parts, percents, ratios and the like are by weight.

In Examples 1 to 6, gas chromatographic analysis was carried out under the following conditions.

### GC Analysis Conditions

Column: DB1 (J&W) (30m capillary column, inside diameter 0.32mm)
Carrier: helium 3 cc/minute
Split ratio: 40
Detector: FID
Analysis conditions: injection at 300°C 100°C (10 minutes) → 15°C/minute (elevation) → 280°C (8 minutes) detection at 300°C

### Example 1

A mixture containing p-cyanobenzoyl chloride (16.6 g), p-cyanobenzoic acid (14.7 g), and dimethylformamide (0.15 g) was stirred vigorously at 120°C, and phosgene gas (20 g) was introduced into the mixture over 90 minutes. After dry nitrogen gas was introduced into the reaction mixture for one hour, the mixture was distilled under reduced pressure, to thereby obtain 32.5 g of p-cyanobenzoyl chloride (yield 98%, based on p-cyanobenzoic acid; bp. 110°C/2 mmHg). Gas chromatographic analysis revealed that the obtained p-cyanobenzoyl chloride had a purity of 99% or more.

### Example 2

A mixture containing p-cyanobenzoyl chloride (16.6 g), p-cyanobenzoic acid (14.7 g), imidazole (0.2 g), and xylene (150 ml) was stirred vigorously at 140°C, and phosgene gas (20 g) was introduced to the mixture over two hours. After dry nitrogen gas was introduced to the reaction mixture for one hour, the mixture was distilled under reduced pressure, to thereby obtain 31.8 g of p-cyanobenzoyl chloride (yield 96%, based on p-cyanobenzoic acid). The p-cyanobenzoyl chloride obtained had a purity of 99% or more.

### Example 3

p-Cyanobenzoic acid (14.7 g) and acetonitrile (300 ml) were mixed and stirred vigorously while the solvent was refluxed, and phosgene gas (30 g) was introduced to the mixture over four hours. After dry nitrogen gas was introduced to the reaction mixture for one hour, acetonitrile was removed under ambient pressure, and was further removed under reduced pressure through distillation, to thereby obtain 16.2 g of p-cyanobenzoyl chloride (yield 98%, based on p-cyanobenzoic acid). The p-cyanobenzoyl chloride obtained had a purity of 99%.

### Example 4

A mixture containing m-cyanobenzoyl chloride (16.6 g) and m-cyanobenzoic acid (14.7 g) was stirred vigorously at 130°C, and phosgene gas (20 g) was introduced to the mixture over two hours. After dry nitrogen gas was introduced to the reaction mixture for one hour, the mixture was distilled under reduced pressure, to thereby obtain 31.8 g of m-cyanobenzoyl chloride (yield 96%, based on m-cyanobenzoic acid; bp. 127°C/11 mmHg). The m-cyanobenzoyl chloride obtained had a purity of 99% or more.

### Example 5

A mixture containing pyridine (8.7 g) and dichloromethane (150 ml) was stirred vigorously at room temperature. A solvent wherein phosgene (10.4 g) was dissolved in dichloromethane (150 ml) was added to the mixture, and stirred. p-Cyanobenzoic acid (14.7 g) was added to the reaction mixture, and stirred vigorously at room temperature for one hour. Gas chromatographic analysis revealed that the p-cyanobenzoyl chloride obtained had been produced at a yield of 98% and had a purity of 99% or more.

### Example 6

A mixture containing m-cyanobenzoic acid (14.7 g), a phosgene trimer (14.8 g), pyridine (11.9 g), and 1,2-dichloroethane (300 ml) was stirred vigorously at 90°C for four hours. The reaction mixture was cooled, and gas chromatographic analysis revealed that the m-cyanobenzoyl chloride obtained had been produced at a yield of 99% based on m-cyanobenzoic acid and had a purity of 99% or more.

As described hereinabove, the present invention provides a process for producing a cyanobenzamide compound with high selectivity and yield, which process comprises hydrolyzing one nitrile group of an easily available phthalonitrile compound.

The invention also provides a process for producing a cyanobenzoic acid compound and a cyanobenzoate ester compound without affecting the cyano group of the benzene ring, which process comprises transforming, under acidic conditions, the thus-produced cyanobenzamide compound serving as a starting material into the target compounds.

The invention also provides a process for producing an alkyl cyanobenzoate compound with high selectivity and yield, which process comprises reacting a phthalonitrile compound serving as a starting material with an aliphatic alcohol in the presence of an acid, to thereby selectively transform one nitrile group into an imino ether group.

The invention also provides a process for producing a cyanobenzoic acid compound without affecting the cyano group of the benzene ring, which process comprises oxidizing a cyanobenzylamine compound serving as a starting material.

The invention also provides a process for producing a cyanobenzoic acid compound with high selectivity and yield without affecting the cyano group of the benzene ring, which process comprises hydrolyzing an alkyl cyanobenzoate compound.

The invention also provides a process for producing a cyanobenzoic chloride compound without affecting the cyano group of the benzene ring, which process comprises chlorinating a cyanobenzoic acid compound serving as a starting material.

The cyanobenzoic acid compound, cyanobenzamide compound, alkyl cyanobenzoate compound, and cyanobenzoyl chloride compound produced through the process according to the present invention are useful intermediates for a variety of chemicals such as pharmaceuticals, agrochemicals, liquid crystals, and monomers for functional polymers.

## Claims

1. A process for producing a cyanobenzoyl chloride compound represented by the following formula: 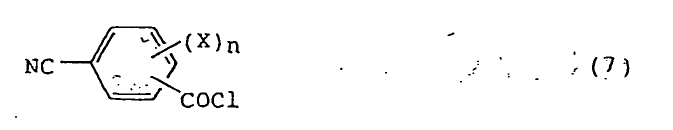 wherein each of -COCl and -X represents a substituent of the benzene ring; the -COCl group is bonded to an m- or the p-position with respect to the CN group; X represents a chlorine atom or a fluorine atom; n is an integer between 0 and 4 inclusive; and when n is 2 or more the plurality of X's may be identical to or different from one another, which process comprises
reacting a cyanobenzoic acid compound represented by the following formula: 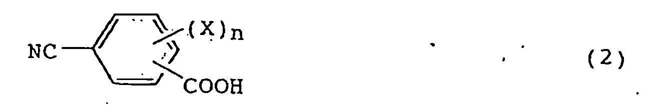 wherein -X represents a substituent of the benzene ring with phosgene or a precursor thereof, the precursor being a substance convertible to phosgene with heat or through a treatment with a catalyst.

2. A process for producing a cyanobenzoyl chloride compound according to Claim 1, wherein the reaction is carried out in the presence of a cyanobenzoyl chloride compound corresponding to the cyanobenzoic acid compound.

3. A process for producing a cyanobenzoyl chloride compound according to Claim 1, wherein the reaction is carried out in the presence of a tertiary amide compound or a tertiary amine compound.

4. A process for producing a cyanobenzoyl chloride compound according to any one of Claims 1 to 3, wherein the cyanobenzoic acid compound represented by formula (2) is m- or p-cyanobenzoic acid, and the cyanobenzoyl chloride compound represented by formula (7) is m- or p-cyanobenzoyl chloride.

5. A process for producing a cyanobenzoyl chloride compound according to any one of Claims 1 to 4, wherein phosgene is used in an amount of 1.1 - 4 mol per 1 mol of the cyanobenzoic acid compound.

## Patentansprüche

1. Verfahren zur Herstellung einer Cyanobenzoylchloridverbindung der folgenden Formel 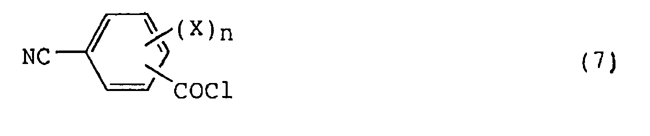 wobei -COCl und -X jeweils einen Substituenten des Benzolrings darstellen; die -COCl-Gruppe bezüglich der CN-Gruppe an einer m- oder der p-Position gebunden ist; X ein Chloratom oder ein Fluoratom darstellt; n eine ganze Zahl zwischen 0 und einschließlich 4 ist; und, wenn n 2 oder mehr ist, die Mehrzahl der X zueinander identisch oder voneinander verschieden sein kann,
wobei das Verfahren umfasst,
Umsetzen einer Cyanobenzoesäureverbindung der folgenden Formel: 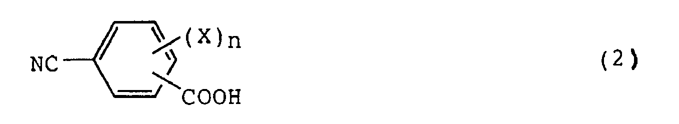 wobei -X einen Substituenten des Benzolrings mit Phosgen oder einem Vorläufer davon darstellt, wobei der Vorläufer eine mittels Wärme oder mittels Behandlung mit einem Katalysator in Phosgen umwandelbare Substanz ist.

2. Verfahren zur Herstellung einer Cyanobenzoylchloridverbindung nach Anspruch 1, wobei die Reaktion in Gegenwart einer der Cyanobenzoesäureverbindung entsprechenden Cyanobenzoylchloridverbindung durchgeführt wird.

3. Verfahren zur Herstellung einer Cyanobenzoylchloridverbindung nach Anspruch 1, wobei die Reaktion in Gegenwart einer tertiären Amidverbindung oder einer tertiären Aminverbindung durchgeführt wird.

4. Verfahren zur Herstellung einer Cyanobenzoylchloridverbindung nach einem der Ansprüche 1 bis 3, wobei die Cyanobenzoesäureverbindung der Formel (2) m- oder p-Cyanobenzoesäure ist, und die Cyanobenzoylchloridverbindung der Formel (7) m- oder p-Cyanobenzoylchlorid ist.

5. Verfahren zur Herstellung einer Cyanobenzoylchloridverbindung nach einem der Ansprüche 1 bis 4, wobei Phosgen in einer Menge von 1,1 - 4 Mol pro 1 Mol der Cyanobenzoesäureverbindung verwendet wird.

## Revendications

1. Processus de production d'un composé chlorure de cyanobenzoyle représenté par la formule suivante : 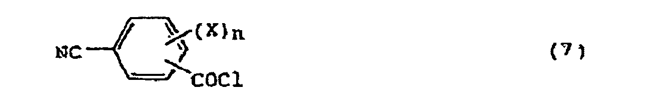 dans laquelle chacun de -COC1 et de -X représente un substituant du cycle benzène ; le groupe -COCl est lié à une position m ou à la position p par rapport au groupe CN ; X représente un atome de chlore ou un atome de fluore ; n est un nombre entier de 0 à 4 inclus ; et lorsque n est 2 ou plus, la pluralité des X peuvent être identiques ou différents les uns des autres,
lequel processus comprend
le fait de faire réagir un composé acide cyanobenzoïque représenté par la formule suivante : 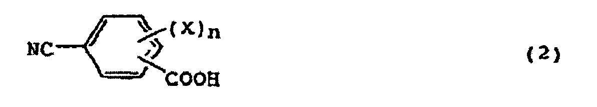 dans laquelle -X représente un substituant du cycle benzène avec un phosgène ou un précurseur de celui-ci, le précurseur étant une substance convertible en phosgène par chauffage ou par un traitement avec un catalyseur.

2. Processus de production d'un composé chlorure de cyanobenzoyle selon la revendication 1, dans lequel la réaction est réalisée en présence d'un composé chlorure de cyanobenzoyle correspondant au composé acide cyanobenzoïque.

3. Processus de production d'un composé chlorure de cyanobenzoyle selon la revendication 1, dans lequel la réaction est réalisée en présence d'un composé amide tertiaire ou d'un composé amine tertiaire.

4. Processus de production d'un composé chlorure de cyanobenzoyle selon l'une quelconque des revendications 1 à 3, dans lequel le composé acide cyanobenzoïque représenté par la formule (2) est l'acide m- ou p-cyanobenzoïque, et le composé chlorure de cyanobenzoyle représenté par la formule (7) est le chlorure de m- ou p-cyanobenzoyle.

5. Processus de production d'un composé chlorure de cyanobenzoyle selon l'une quelconque des revendications 1 à 4, dans lequel le phosgène est utilisé en une quantité de 1,1 à 4 moles par mole du composé acide cyanobenzoïque.
